Europäisches Patentamt

European Patent Office

Office européen des brevets

(19)

(11) Publication number: **0 337 074**
**A1**

# (12) EUROPEAN PATENT APPLICATION

(21) Application number: **89102559.5**

(22) Date of filing: **15.02.89**

(51) Int. Cl.⁴: **A61M 5/315**

(30) Priority: **11.04.88 DE 3811973**

(43) Date of publication of application:
**18.10.89 Bulletin 89/42**

(84) Designated Contracting States:
**ES**

(71) Applicant: **Greive, Michael**
**Urbanstrasse 12**
**D-4403 Senden-Ottmarsbocholt(DE)**

(72) Inventor: **Greive, Michael**
**Urbanstrasse 12**
**D-4403 Senden-Ottmarsbocholt(DE)**

(74) Representative: **Sternagel, Hans-Günther, Dr.**
**et al**
**Patentanwälte Dr. Michael Hann Dr. H.-G.**
**Sternagel Sander Aue 30**
**D-5060 Bergisch Gladbach 2(DE)**

(54) **Disposable hypodermic syringe.**

(57) A hypodermic syringe which permits only a single use has a plunger (1) which can be pushed into a sleeve and has webs (3,4,4a) situated opposite one another in pairs on the plunger rod (2). These webs (3,4,4a) cooperate with a stop ring (6) placed on or integrated into the grip plate (15) of the sleeve of the hypodermic syringe and, by virtue of the formation of grooves (5) and detent noses (8,9) in the central opening (7) of the stop ring (6), permit only three movements: drawing up of injection material, rotation of the plunger (1) about its longitudinal axis and ejection of the injection material from the syringe.

EP 0 337 074 A1

## Disposable hypodermic syringe

The invention relates to a hypodermic syringe which, after a single use, can no longer be used, in particular to a locking device which, in cooperation with the specially designed plunger, ensures that a multiple use of the hypodermic syringe is not readily possible.

Hypodermic syringes for single use are in principle known, but need to be deliberately rendered inoperative or destroyed after use.

German Utility Model 88 04 656.7 describes a hypodermic syringe in which a detent member arranged in the barrel of the syringe is in engagement with the plunger. A toothing which cooperates with the detent member is formed on the central piece of the plunger, with the result that only a single use is possible.

DE-A-28 10 370 discloses a syringe in which the plunger can be locked in various stroke positions in the syringe barrel via catches. The plunger has a toothed rod in the form of a cross which engages in detent projections of a locking plate on the barrel. Locking is accomplished by rotating the barrel about its longitudinal axis, the toothed rod thereby coming into engagement with the locking plate or the engagement being released again.

It is the object of the invention to create a device which can be combined with known hypodermic syringes and ensures that a multiple use of the hypodermic syringe is not possible.

This object is achieved by a hypodermic syringe having a cylindrical sleeve and a connection part for a cannula at one side and a grip plate at the rearward end of the sleeve, having a plunger which can be pushed into the sleeve and the plunger rod of which has along its length 2 pairs of guide webs arranged at right angles to one another, opposite one another, projecting radially from the plunger rod and having along their length grooves arranged at a short distance from one another, and there being a stop plate which can be fitted onto the grip plate of the sleeve, the stop plate having a concentrically arranged opening for the plunger rod, there being in the opening detent noses for engagement in the grooves of the webs on the plunger rod, which is characterized in that a) the first webs are of shorter configuration in the radial direction than the second webs and the first webs are provided along their length with grooves, and b) the detent noses in the opening of the stop plate are, at the rim of the opening, a first detent nose extending radially inwards and directed obliquely upwards and, at the rim, arranged at an angle of about 135° to the first detent nose, a second detent nose, directed radially inwards and obliquely downwards, for the purpose of engagement in the grooves of the first webs on the plunger rod, and, at an angle of in each case 80-100° to the second detent nose, the diameter of the opening is reduced by webs projecting into the opening, and there is between the webs a groove or an intermediate space of a depth such that, in the intermediate space, the diameter of the opening is not reduced.

The cross-section of the stop plate is preferably C-shaped and has a narrow slot between the outer rim and the central opening, with the result that it can be pushed onto the grip plate of a preassembled syringe and the plunger rod of the syringe plunger extends through the opening in the stop plate. The stop plate is held on the grip plate by means of a clamping fit, the bent-round legs of the C-profile gripping the rim of the grip plate of the sleeve of the hypodermic syringe. It is particularly advantageous if the shape of the circumference of the stop plate corresponds to the shape of the grip plate of the hypodermic syringe sleeve. This ensures the pushing-on and clamping of the stop plate in a predetermined position on the grip plate of the sleeve. For better anchoring of the stop plate on the grip plate it is advantageous if, on each side of the sleeve, the grip plate has a groove or depression or through opening into which detent noses in each case arranged on the legs of the C-profile of the stop plate can engage. The position of the grooves in the grip plate and of the detent noses on the stop plate is such that they are aligned with one another and engagement is possible.

It is also advantageous if the outer circumference of the stop plate is greater than the grip plate of the sleeve, so that bending round in a bead formation can be carried out for the purpose of attachment. In this case, the outer rim of the stop plate grasps the grip plate of the sleeve along its periphery after assembly and removal is no longer possible without destruction. A firm connection between the stop plate and the grip plate of the syringe sleeve can also be achieved if the grip plate has two holes and the stop plate has two downward-directed pegs, the position of which corresponds to the bores in the grip plate, so that the pegs can engage in the bores. A firm fit can be obtained by welding or glueing the pegs or by enlarging the ends of the pegs.

However, it is also possible to integrate the stop plate into the sleeve of the syringe in the vicinity of the grip plate or into the grip plate itself. For example such that the grip plate has the detent noses and webs or segments required for locking in the central opening or a flat stop plate is glued or welded onto the grip plate. In the case of integration into the sleeve, the sleeve wall forms the plate having the central opening and the detent noses and segments or webs extend from the inside wall of the sleeve into the interior of the sleeve.

It is particularly advantageous if the stop plate is produced together with the syringe plunger, for example by injection moulding using a correspondingly designed mould. In this mode of production, the plunger rod already extends through the opening of the stop plate and is initially connected to the plunger rod by a thin destructible layer of plastic or a film. In such a case, the slot connection of the central opening of the stop plate to the rim, which connection facilitates fitting onto the plunger rod, can be dispensed with. Combined parts of this kind can be produced by injection moulding. During the assembly of the syringe and after the pushing of the plunger into the sleeve of the syringe, the breakable connection, stemming from their common production, between plunger rod and stop plate is severed and the stop plate is attached to the syringe by bending the edge around the grip plate in a bead formation. However, attachment can also be effected by welding, glueing or by means of the engagement of pegs on the stop plate in holes in the grip plate of the sleeve. In these cases, the stop plate preferably has the same shape and the same outer circumference as the grip plate of the syringe sleeve.

In the preassembled condition, the syringe plunger is introduced into the sleeve of the hypodermic syringe in such a way that the plunger plate is situated at the forward end of the sleeve and, in the axial direction of the sleeve, the plunger rod is aligned such that one of the first webs on the plunger rod exhibiting grooves is in engagement with the first detent nose of the stop plate. Despite engagement in the grooves, the upward-directed first detent nose permits the plunger to be pulled out for the purpose of drawing up injection fluid into the syringe. The grooves are preferably formed obliquely and oriented such that the open grooves permit the detent noses to slide over the flanks of the grooves. In this position, it is not possible to press the plunger into the sleeve because, in the event of movement counter to the orientation of the upward-directed detent nose, said detent nose is pressed into the grooves and thereby blocks further movement. In order, on the one hand, to facilitate the pulling out of the plunger and, on the other hand, to reinforce the blocking effect against pressing in, the formation of a sawtooth profile for the grooves is preferred, the opening of the grooves extending obliquely towards the forward end of the plunger rod. The rounded flanks of the sawteeth in each case face the rearward end of the plunger rod and the sharp flank provided for blocking engagement with the detent nose faces the forward end of the plunger rod.

By means of a rotation of the plunger about its longitudinal axis, the engagement of the first detent nose in the grooves of the first web can be released. Upon rotation by 45°, said rotation being possible in only one direction by virtue, in particular, of the design of the flanks on the segments or webs extending into the opening of the stop plate, the mutually opposite second webs on the plunger rod come into engagement with the grooves or move into the intermediate space between the webs projecting into the opening of the stop plate and block the plunger rod against any further rotation about its longitudinal axis. These segments or webs, which reduce the diameter of the opening of the stop plate, starting from the rim, are offset by 45° and 225° respectively with respect to the first detent nose on the rim of the opening. It is also possible to form a web with a groove at only one location on the rim of the opening or to form a pair of webs with an intermediate space.

By virtue of the rotation of the plunger rod by 45°, the web on the plunger rod which is opposite the released first web on the plunger rod simultaneously also comes into engagement with the second downward-directed detent nose in the opening of the stop plate, said detent nose being offset by about 135° with respect to the first detent nose. In this position, the plunger can be moved into the sleeve for the purpose of ejecting the material to be injected but cannot be pulled out again because then the detent nose presses into the grooves of this web. In order to facilitate injection, the grooves are preferably made to extend obliquely or to form a sawtooth profile, the opening of the grooves extending obliquely towards the rearward end of the plunger rod. The rounded flanks of the sawteeth in each case face the forward end of plunger rod and the sharp flanks provided for blocking engagement with the detent nose face the rearward end of the plunger rod.

In order to facilitate the preassembly of the syringe, the first webs on the plunger rod in one embodiment of the invention have the same length in the radial direction and the grooves in the first webs arranged opposite one another on the plunger rod have the same shape. The first and second detent nose of the stop plate extend an equal distance into the opening of the stop plate. The only difference between the first and second detent noses offset at an angle of 135° with respect to one another on the rim of the opening is that the first detent nose extends obliquely to one side from the stop plate fitted on the grip plate and the second detent nose extends obliquely to the other side from the stop plate in order in each case to permit the movement of the plunger in only one direction. The first detent nose preferably extends upwards, i.e. in that direction in which the stop plate faces away from the grip plate, and the second detent nose extends downwards, i.e. towards the grip plate on the sleeve of the hypodermic syringe.

In another embodiment, the grooves in the first webs are once again preferably arranged obliquely to form a sawtooth profile, the direction of the sawteeth on the mutually opposite first webs preferably being different, so that, during the preassembly of the hypodermic syringe, the plunger has to be introduced in a particular position into the sleeve, namely in such a way that, when the stop plate is subsequently fitted onto the grip plate of the sleeve, the first web comes into engagement by the sawtooth grooves open obliquely towards the forward end of the plunger rod with the first detent nose of the stop plate. In order to exclude faulty preassembly, the first webs situated opposite one another on the plunger rod can have different extension in the radial direction, the first and second detent nose on the stop plate then also simultaneously projecting different distances into the opening of the stop plate. For example, such that the first detent nose projects somewhat further into the opening of the stop plate than the second detent nose.

Accordingly, the first web on the plunger rod, said web being provided for engagement with the first detent nose, is of shorter configuration in the radial direction than the opposite web, which is provided for engagement with the second detent nose, which does not project as far into the opening.

The extent of the first webs situated opposite one another on the plunger rod in the radial direction is in each case matched to the length of the first and second detent noses projecting into the opening of the stop plate in such a way that the detent noses engage in the grooves when the plunger rod is introduced in a corresponding position into the sleeve or is rotated within the sleeve.

By virtue of the formation according to the invention of four webs on the plunger rod, in each case arranged in pairs opposite one another, a first pair of webs having detent grooves, and the two detent noses projecting into the opening of the stop plate and one or two further webs projecting into the opening from the rim, which in each case occupy a circular segment on the rim of the opening and in each case have a groove or an intermediate space for engagement with the second pair of webs on the plunger rod, it is ensured that, when the stop plate has been fitted on, the hypodermic syringe can be drawn up and emptied only once. Instead of a circular segment with a groove, it is also possible for there to be a pair of webs with an intermediate space. In order to make possible the single use, it is necessary that, in the preassembled condition, the syringe should contain the plunger with the plunger provided with webs in such a position that, after the stop plate has been fitted, one of the first webs is in engagement with the first detent nose. In this position, the only movement possible, apart from a rotary movement about the longitudinal axis, is the pulling out of the plunger for the purpose of drawing up injection material into the syringe. If the plunger is then rotated about its longitudinal axis, the engagement of the first detent nose in the grooves of the first web is released, the second detent nose simultaneously comes into engagement with grooves of the first webs and, by engagement of the second webs in corresponding grooves in the segments or intermediate space between webs in the opening of the stop plate, the plunger is blocked against any further rotary movement. Thereafter, only the ejection of injection material by pressing the plunger into the sleeve is still possible.

For reasons of cost, disposable articles for medical use are preferably produced from plastic. Suitable plastics for the sleeve, the plunger having the special plunger rod, and the stop plate are thermoplastics or thermosets, thermoplastics being preferred because they can be deformed more easily during the production of the parts. The suitable plastics include polyethylene, polypropylene, polyvinyl chloride, polyacetals, polystyrenes, polyamides, polyurethanes, polycarbonates, polyesters and copolymers of the homopolymers mentioned. In principle, however, it is also possible to produce the stop plate from metal, for example by punching it out from sheet metal and forming the rim into a C-profile and bending the detent noses in the desired direction.

The solution according to the invention also includes a process for producing the plunger and the stop plate simultaneously and in common. In this process, the plunger for a hypodermic syringe having a plunger rod, a plunger at one end of the plunger rod and a grip plate at the other end of the plunger rod is produced from a thermoplastic by injection moulding in one mould. The characterizing feature of this process consists in the fact that the plunger rod has along its length two pairs of guide webs arranged at right angles to one another, opposite one another, projecting radially from the plunger rod and having along their length grooves arranged at a short distance from one another, and in that a stop plate having a concentrically arranged opening through which the plunger rod extends is simultaneously produced which is initially connected to the plunger rod by a destructible film arising from production, and in that, in the opening, the stop plate has detent noses for engagement in the grooves of first webs of the plunger rod and webs for engagement with second webs of the plunger rod.

Decisive for the solution on the object according to the invention is the structural configuration, which makes possible the cooperation of the stop plate with the radially extending webs arranged on the plunger rod of the injection plunger, such that only the three movements already described above of the plunger in the sleeve are possible. The drawing up of injection material by pulling out the plunger is accomplished in the customary manner, one of the first webs being in engagement with the first detent nose on the stop plate. In order to make an ejection of injection material possible,a rotary movement of the plunger about the longitudinal axis of the plunger rod by 45° is required after the syringe has been pulled up, in order to cause the second webs of the plunger rod to engage in the mutually opposite grooves or the intermediate space of a pair of webs at the rim of the opening of the stop plate and to block the plunger against any further rotary movement on about the axis. Simultaneously with the cancellation of the engagement of the first detent nose, the engagement of the plunger rod with the second detent nose is brought about by the rotary movement, said engagement permitting the plunger to be pressed into the sleeve but not pulled out of the sleeve. After the emptying of the hypodermic syringe, the plunger is locked against any further rotary or stroke movement as long as the stop plate is on the grip plate of the sleeve.

In order to make the removal of the stop plate from the grip plate of the sleeve more difficult, the bent-round rim of the C-profile and the grip plate can be provided with projections and catches which engage in one another by snapping in and can no longer be parted without destroying the parts. It is particularly preferable to bend the outer rim of the stop plate around the grip plate in a bead formation along the entire circumference in order to make detachment impossible without destruction. Grip plate and stop plate can also be glued or welded together. In order to render impossible multiple use by overturning the plunger rod, it is preferable to provide the plunger rod with a predetermined breaking point in the vicinity of the rearward end, for example by a reduction in cross-section on a short piece. In the case of improper, in particular undesired, rotation of the plunger about its longitudinal axis, the plunger rod is sheared off and the syringe thereby becomes unusable.

The invention is now explained in greater detail with reference to the figures.

Fig. 1 shows, in perspective view, the design of the first and second webs on the plunger rod of the plunger of a hypodermic syringe.

Fig. 2 shows the stop plate in plan view.

Fig. 3 shows the stop plate in section along the line A - A of Fig. 2 and shows the C-profile-shaped cross-section.

Fig. 4 shows the position of the plunger rod of the syringe in the preassembled condition with the webs when being passed through the opening of the stop plate for the drawing up of injection material into the syringe.

Fig. 5 shows the position of the plunger rod with the webs when being passed through the opening of the stop plate, following the rotary movement, for the purpose of ejecting the injection material from the syringe.

Fig. 6 shows a stop plate anchored on the grip plate on the syringe sleeve, in section along the line A - A of Fig. 7.

Figs. 7 and 8 show the embodiment of the stop plate of Fig. 6 from above.

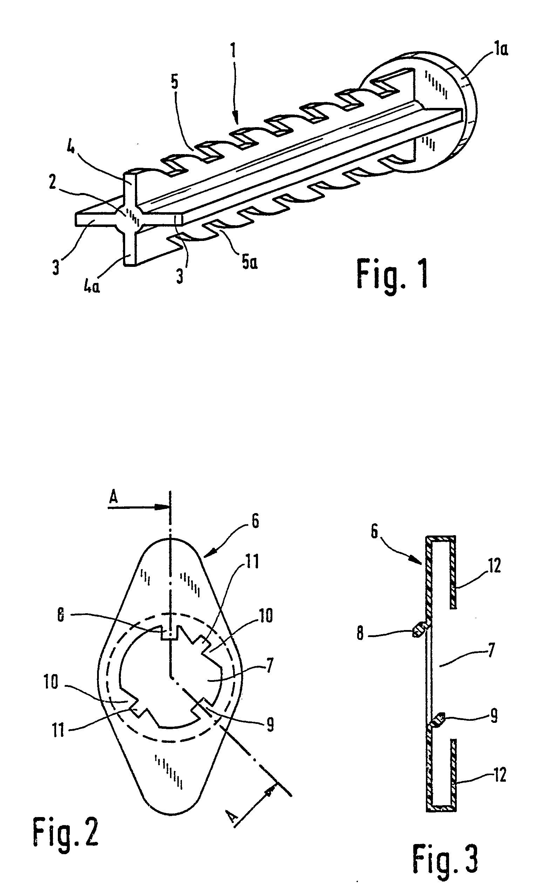

The plunger 1 for a hypodermic syringe, said plunger being represented in part in Fig. 1, has a plunger rod 2 extending from the plunger plate 1a to the rearward end having a grip plate (not shown). From the plunger rod 2 there extend along its length, in the radial direction, first webs 4, 4a and, at a right angle thereto, second webs 3, which are arranged pair-wise opposite one another. In the radial direction, the second webs 3 have a greater width than the first webs 4, 4a. The distance of the outer edges of the pair 3 of webs from one another is less than the inside diameter of the sleeve of the syringe but greater than the diameter of the opening of the stop plate (shown in another figure) at the locations exhibiting the first and second detent noses. The first webs 4 and 4a are provided with grooves 5, 5a, preferably obliquely extending grooves or a sawtooth profile. The grooves 5, 5a in each case extend obliquely to the longitudinal axis of the plunger rod 2, the grooves 5 and 5a in each case opening in the opposite direction. In the case of the grooves 5, the sharp flank faces the rearward end, while, in the case of the grooves 5a, the sharp flank faces the forward end of the plunger 1.

Fig. 2 shows the stop plate 6 designed to engage with the webs of the plunger rod, from above. The external shape, i.e. the circumference, corresponds to the shape of the grip plate on sleeves of hypodermic syringes. The stop plate 6 has a central opening 7, the diameter of which corresponds to the inside diameter of the sleeve of the hypodermic syringe or is somewhat smaller than the latter. In order to facilitate the introduction of the plunger rod 2 into the opening 7, there can be a slot connection (not shown) between the opening 7 and the outer rim of the stop plate 6. Two first and second detent noses 8 and 9, offset by 135° with respect to one another, extend radially from the rim of the opening 7 of the stop plate 6 towards the centre. These detent noses have a length such that engagement in the grooves of the first webs on the plunger rod occurs when the plunger rod is moved through the opening 7 of the stop plate. At an angle of about 40-50° to the first detent nose 8 within the angle of about 135° and at an angle of 130-140° to the first detent nose to the other side, between the two detent noses 8 and 9, the diameter of the opening 7 is reduced by two mutually opposite segments or webs 10 extending towards the centre into the opening, the segments each having in their centre, i.e. at an angle of 45° to 225° to the first detent nose 8 in each case one groove 11, the depth of which is such that, in the region of the grooves 11, the diameter of the opening corresponds approximately to the inside diameter of the sleeve. The flanks of the webs are formed obliquely with respect to the rim of the opening and are flattened in such a way that the webs produce a circular segment which extends at the rim of the opening and that, when the plunger is rotated about its longitudinal axis in the sleeve, the second webs 3 on the plunger rod 2 can slide over the flanks of the webs 10 and engage in the grooves 11 and block the plunger against any further rotatary movement about its longitudinal axis.

Fig. 3 shows the stop plate 6 in section, allowing the oblique position of the first detent nose 8 to the one side and that of the second detent nose 9 to the other side to be seen. The opening 7 is situated in the centre. In order to be able to secure the stop plate 6 on the grip plate of a syringe sleeve, it has a C-shaped cross-section. When pushed on, the bent-round legs 12 of the C grip the rim of the grip plate of the sleeve. However, the detent noses can also be arranged in the plane of the stop plate within the opening 7.

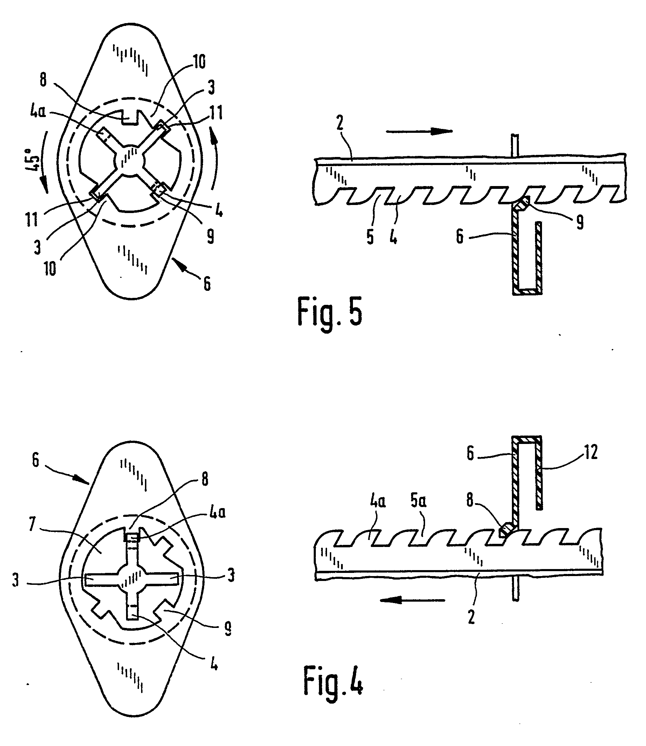

In Fig. 4, the cooperation of the webs 3, 4, 4a, during the pulling up of the hypodermic syringe, with the stop plate 6 fitted onto the grip plate of the sleeve is shown. The first, radially extending web 4a fixed to the plunger rod 2 and having the grooves 5 is in engagement with the first detent nose 8, which projects obliquely upwards into the opening 7 of the stop plate 6. In the case of a stroke movement of the plunger in the direction of the arrow, the detent nose 8 slides over the round flanks of the grooves 5a in the first web 4a. The plunger is prevented from being pushed into the sleeve of the syringe (movement counter to the direction of the arrow) by the blocking engagement of the first detent nose 8 in the grooves 5a. The other first web 4 and the second webs 3 are free within the sleeve and the opening 7 of the stop plate, as is the second detent nose 9 of the stop plate 6.

When the drawing up of injection material into the hypodermic syringe is complete, the plunger is rotated about its longitudinal axis into the position shown in Fig. 5. The angle of rotation amounts to 45° outside the angle of about 135° between the two detent noses in the direction of the second detent nose 9 (see direction arrow in the figure). The engagement of the first detent nose 8 with the first web 4a is thereby cancelled, but the opposite first web 4 is brought into engagement with the second detent nose 9. At the same time, the second webs 3 snap into the grooves 11 of the segments 10 and block the plunger against any further rotary movement about its longitudinal axis. The pressing in of the plunger rod 2 into the sleeve of the hypodermic syringe is facilitated by the fact that the second detent nose 9 can slide over the flanks of the grooves 5 in the web 4. If an attempt is made to move the plunger in the opposite direction, namely to pull it out of the sleeve, the detent nose 9 is pressed into the grooves 5 by virtue of its oblique position and blocks any such intended movement.

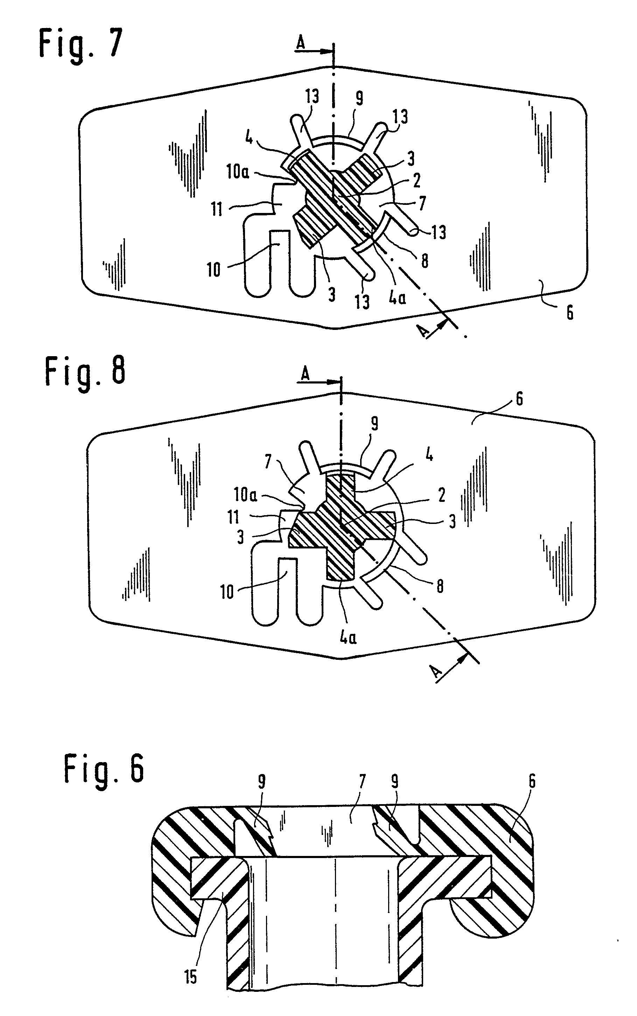

Fig. 6 shows the preferred attachment of the stop plate 6 on the grip plate 15 of the syringe sleeve by bending the outer rim of the stop plate 6 in a bead formation around the grip plate 15. In this embodiment, the detent noses 8 and 9 are arranged in the plane of the stop plate 6 and project into the central opening 7. This arrangement in the plane of the stop plate has the advantage that the undesired removal of the detent noses is made more difficult. In this configuration, the detent noses can be formed directly during the production of the stop plate 6 from plastic, at the same time as the latter.

Figs. 7 and 8 show the embodiment of the stop plate 6 represented in Fig. 6, from above. The plunger rod 2 has first webs 4 and 4a and second webs 3. The detent noses 8 and 9 project into the opening 7. The webs 10 and 10a, which are offset by a certain angle with respect to the first detent nose 8, reduce the diameter of the opening 7 at certain locations. To facilitate the formation of the detent noses 8 and 9, narrow recesses 13 are in each case formed next to them. Fig. 7 shows the cooperation of the webs 3, 4, 4a with the stop plate 6 fitted onto the grip plate when the syringe is pulled up. The web 4a extending radially from the plunger rod 2 is in engagement with the first detent nose 8, which points upwards, with the result that the plunger rod 2 can be pulled out but cannot be pushed in, because the detent nose 8 in engagement with the web 4a blocks such a movement. After the syringe has been pulled up, the plunger rod 2 is rotated about its longitudinal axis into the position shown in Fig. 8. The engagement of the web 4a in the detent nose 8 is released, while the web 4 is in engagement with the detent nose 9. In this position, the plunger rod 2 can be pressed into the syringe sleeve to eject the injection material. The cooperation of the web 4 with the downward-directed detent nose 9 blocks the plunger rod 2 from being pulled out again. The web 10a shown in Fig. 7 projects into the opening 7 and blocks the rotation of the plunger rod 2 about its longitudinal axis in an undesired direction by the fact that the web 4 rests against the web 10a. Following the rotation of the plunger rod into the position shown in Fig. 8, the web 3 protruding from the plunger rod 2 is situated between the webs 10 and 10a projecting from the rim into the opening 7, said webs blocking any further rotary movement of the plunger rod 2 about the longitudinal axis, with the result that the engagement of the web 4 with the detent nose 9 can no longer be released. In order to facilitate the snapping in of the web 3 into the intermediate space 11 between the webs 10 and 10a, one of the flanks of the web 3 in this embodiment is bevelled.

Example

A syringe sleeve having a length of 87.0 mm, an outside diameter of 6.0 mm, an inside diameter of 4.8 mm and a grip plate measuring 10 x 19 mm is produced by injection moulding using a correspondingly designed mould from elastomer-modified polypropylene (Vestolen P 7000, Hüls) or another suitable thermoplastic. For injection moulding, a Battenfeld machine 230/45 is used, at a temperature of 240° C, an injection pressure of 80/40 bar and a cycle time of 12-16 sec. The mould has 8 cavities and the mould clamping pressure is 220 Nm.

Other suitable thermoplastics should have a melt flow index (MFI) 190/5 of about 4 g/10 min. and the modulus of elasticity should be about 1300 N/mm$^2$ (150 178).

The plunger and the stop plate are produced from a polyamide (Ultramid A3K, BASF) on the same injection moulding machine using a mould likewise having 8 cavities. The length of the plunger rod is 90 mm. The webs on the plunger rod result in a plunger diameter of 4.7 mm. The notches are 0.6-0.8 mm deep and 1.2-1.4 mm long.

The processing temperature is 230° -300° C, the mould temperature is 80° -100° C, the injection pressure is 80-100 bar/40 bar and the cycle time is 14-20 sec.

The hypodermic syringe is assembled as follows. An annular seal of rubber is fitted onto the plunger rod in order to seal the plunger plate. The stop plate is placed on the plunger rod and the plunger is introduced into the sleeve. The outer circumference of the stop plate is 1-2 mm greater than the grip plate of the sleeve, with the result that, in the plasticized condition, the rim can be bent in a bead formation round the grip plate. The preassembled syringe is generally packed under sterile conditions and, when used, is provided with a hypodermic needle.

List of reference numerals

1 Plunger
1a Plunger plate
2 Plunger rod
3 Second webs
4,4a First webs
5,5a Grooves
6 Stop plate
7 Central opening
8 First detent nose
9 Second detent nose
10 Webs, segments
11 Groove, intermediate space
12 Bent-round legs or rim
13 Recesses
15 Grip plate of the sleeve

Claims

1. Hypodermic needle having a cylindrical sleeve and a connection part for a cannula at one side and a grip plate (15) at the rearward end of the sleeve, having a plunger (1) which can be pushed into the sleeve and the plunger rod (2) of which has along its length 2 pairs of guide webs (3, 4, 4a) arranged at right angles to one another, opposite one another, projecting radially from the plunger rod (2) and having along their length grooves (5) arranged at a short distance from one another, there being a stop plate (6) which can be fitted firmly onto the grip plate (15) of the sleeve, the stop plate (6) having a concentrically arranged opening (7) for the plunger rod (2), and there being in the opening (7) detent noses (8, 9) for engagement in the grooves (5) of the webs (4, 4a) on the plunger rod (2), characterized in that (a) the first webs (4, 4a) are of shorter configuration in the radial direction than the second webs (3) and the first webs (4, 4a) are provided along their length with grooves (5), (b) and the detent noses in the opening (7) of the stop plate (6) are, at the rim of the opening (7), a first detent nose (8) extending radially inwards and directed obliquely upwards and, at the rim, offset at an angle of about 135° to the first detent nose (8), a second detent nose (9), directed radially inwards and obliquely downwards, for the purpose of engagement in the grooves (5) of the first webs (4, 4a) on the plunger rod (2), and, at an angle of in each case 80-100° to the second detent nose (9), the diameter of the opening (7) is reduced by webs (10) projecting into the opening (7), and between the webs (10) there is a groove or an intermediate space (11) of a depth such that, in the intermediate space (11), the diameter of the opening (7) is not reduced.

2. Hypodermic syringe according to Claim 1, characterized in that, in the assembled condition, the outer rim of the stop plate (6) grasps the grip plate (15) of the sleeve along its circumference.

3. Hypodermic syringe according to Claim 1, characterized in that the stop plate (6) has a C-shaped cross-section, whose bent-round C legs, after the stop plate (6) has been fitted onto the grip plate (15) of the sleeve, grip the rim of the grip plate (15).

4. Hypodermic syringe according to Claim 1, characterized in that the grip plate (15) of the sleeve has two holes and the stop plate (6) has two pegs projecting downwards, the positions of the holes and of the pegs being in alignment, with the result that the pegs can engage in the holes.

5. Hypodermic syringe according to Claims 1 to 4, characterized in that the grooves (5) in the first webs (4, 4a) are of sawtooth-shaped design.

6. Hypodermic syringe according to Claim 5, characterized in that the direction of the position of the sawtooth-shaped grooves of the first webs (4 and 4a) is mutually opposite.

7. Hypodermic syringe according to Claims 1 to 4, characterized in that the stop plate (6) is made of metal or of plastic.

8. Hypodermic syringe according to Claim 1, characterized in that, in the vicinity of the rearward end, the plunger rod (2) has a predetermined breaking point in the form of a cross-section reduced on a short piece.

9. Process for producing a plunger for a hypodermic syringe having a plunger rod (2), a plunger at one end of the plunger rod (2) and a grip plate at the other end of the plunger rod (2) from thermoplastic by injection moulding in one mould, characterized in that the plunger rod (2) has along its length two pairs of guide webs (3, 4, 4a) arranged at right angles to one another, opposite one another, projecting radially from the plunger rod (2) and having along their length grooves (5) arranged at a short distance from one another, and in that a stop plate (6) having a concentrically arranged opening (7), through which the plunger rod (2) extends, is simultaneously produced which is initially connected to the plunger rod (2) by a destructible film arising from production, and in that, in the opening (7), the stop plate (6) has detent noses (8, 9) for engagement in the grooves (5) of first webs (4, 4a) on the plunger rod and webs (10) for engagement with second webs (3) of the plunger rod (2).

10. Process according to Claim 9, characterized in that (a) the first webs (4, 4a) are of shorter configuration in the radial direction that the second webs (3) and the first webs (4, 4a) are provided along their length with grooves (5) (b) and the detent noses in the opening (7) of the stop plate (6) are, at the rim of the opening (7), a first detent nose (8) extending radially inwards and directed obliquely upwards and, at the rim, offset at an angle of about 135° to the first detent nose (8), a second detent nose (9), directed radially inwards and obliquely downwards, for the purpose of engagement in the grooves (5) of the first webs (4, 4a) on the plunger rod (2), and, at an angle of in each case 80-100° to the second detent nose (9), the diameter of the opening (7) is reduced by webs (10) projecting into the opening (7), and between the webs (10) there is a groove or an intermediate space (11) of a depth such that, in the intermediate space (11), the diameter of the opening (7) is not reduced.

**Fig. 1**

**Fig. 2**

**Fig. 3**

Fig. 5

Fig. 4

Fig. 7

Fig. 8

Fig. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 208 975 (A. GOMEZ et al.) * Figures 1,3,4; page 4, lines 6,7; page 5, lines 23-31; page 6, lines 11-28 * | 1,4,5 | A 61 M   5/315 |
| A | EP-A-0 209 976 (SHERWOOD MEDICAL COMPANY) * Figures 4-10; page 7, line 26 - page 8, line 1; page 8, lines 5-24 * | 2,3,7 | |
| A | US-A-4 711 637 (LEIGH et al.) * Figures * | 1 | |
| A | US-A-3 934 586 (EASTON et al.) * Figures * | 1 | |
| A | US-A-4 562 844 (CARPENTER et al.) * Figure 2; column 5, lines 41,42 * | 8 | |
| A | WO-A-7 901 111 (Le VEEN et al.) * Figure 1; page 3, lines 14-28 * | 9 | TECHNICAL FIELDS SEARCHED (Int. Cl.4)  A 61 M |

The present search report has been drawn up for all claims

| Place of search | Date of completion of the search | Examiner |
|---|---|---|
| THE HAGUE | 19-07-1989 | SEDY, R. |